# EUROPEAN PATENT APPLICATION

(11) **EP 4 796 060 A1**
(43) Date of publication of application: **26.08.2026**
(21) Application number: 26159429.5
(22) Date of filing: 18.02.2026
(51) Int. Cl.: A61B 17/74, A61B 17/80, A61B 17/82, A61B 17/84, A61B 17/88

(54) **PERIPROSTHETIC HOOK PLATE**

(30) Priority: 19.02.2025 US 202519056879
(71) Applicant: Globus Medical, Inc., Audubon, PA 19403 (US)
(72) Inventor: BORDEAUX, Jean, West Chester, PA 19382 (US); MARKS, Aaron, Philadelphia, PA 19104 (US); KEDZIERSKA, Anna, Conshohocken, PA 19428 (US); MACHEN, Ashley, Phoenixville, PA 19460 (US)
(74) Representative: Morabito, Sara

(57) **Abstract**

Devices, systems, instruments, and methods for promoting healing and stability for bone fractures. The bone stabilization system may include a periprosthetic plate configured to treat a periprosthetic fracture around an existing prosthesis. The periprosthetic plate may be especially suitable for the fixation of fractures and fragments in the greater trochanter region of the proximal femur. The periprosthetic plating may be used to create a rigid construct with permanent fixation to promote primary healing and stability.

## Description

### FIELD

The present disclosure relates to surgical devices, and more particularly, to stabilization systems, for example, for trauma applications.

### BACKGROUND

A periprosthetic fracture is a long bone fracture which occurs adjacent or around an existing prosthesis, typically at the distal or proximal femur. Due to an aging population, the frequency of these types of fractures is increasing. A periprosthetic plate is a type of orthopedic implant used in surgical procedures to stabilize bone or bone fragments around the existing prosthesis, particularly after a fracture or during reconstructive surgery. Periprosthetic fracture plates provide mechanical support and aid in the healing process of bone surrounding the prosthetic device, such as an intramedullary device. The periprosthetic plates may be placed, for example, on the proximal femur, which may be indicated to treat Vancouver Type Ag (e.g., fractures located proximal to the prosthetic stem and involving the greater trochanter), B fractures (e.g., fractures around or just below the stem of the prosthesis), Ag + B (e.g., additional complexity involving the greater trochanter area and the area around or just below the stem of the prosthesis), or other fracture types. Unfortunately, current hook plates are not ideal to treat this indication. Some shortcomings may include: the plates are too thick medial to lateral, which can cause patient discomfort; the plates have insufficient screw options to properly secure the fracture fragments; and the screws are not targeted to engage desired bony anatomy. Thus, there remains a need for improved plate styles and plating systems for the fixation of periprosthetic fractures.

### SUMMARY

To meet this and other needs, and in view of its purposes, the present application provides devices, systems, instruments, and methods for promoting healing and stability for bone fractures, especially periprosthetic fractures. In particular, the periprosthetic plates may be primarily used in cases of periprosthetic fractures, which occur in the bone adjacent to or around an existing joint prosthesis, such as an intramedullary device. These fractures can be a significant complication following joint replacement surgeries, and they require stabilization to ensure proper healing and functionality of the joint. The periprosthetic plates may be especially suitable in treating the greater trochanter region of the proximal femur.

According to one embodiment, a periprosthetic bone plate system includes a bone plate, a cerclage crimp, and a cerclage cable. The bone plate extends along a central longitudinal axis from a proximal end to a distal end configured to sit on bone, a pair of hooks extend from the proximal end, the plate defines a crimp slot having crimp-holding tabs, and cerclage cable holes extend through the plate from the crimp slot to lateral sides of the plate. The cerclage crimp is positionable in the crimp slot such that the crimp-holding tabs retain the cerclage crimp therein. The cerclage crimp have a narrowed body with flared ends, and the cerclage crimp define a pair of cable holes between the flared ends. The cerclage cable is positionable through the plate and the cerclage crimp to secure the plate to the bone.

The periprosthetic bone plate system may include one or more of the following features. The crimp-holding tabs may include a pair of crimp-holding tabs on opposite sides of the crimp slot pointing inward toward one another. Each crimp-holding tab may include a projection defined between two semi-circular cuts. The crimp slot may define a quadrilateral recess sized and dimensioned to receive the cerclage crimp. The plate may define a central linear slot aligned with the central longitudinal axis, and the crimp slot may be positioned along the central linear slot. The cerclage cable holes may include a set of cerclage cable holes with two parallel cable holes on one side and two parallel cable holes on an opposite side of the crimp slot. When the cerclage crimp is positioned within the crimp slot, the cable holes of the cerclage crimp may align with the cerclage cable holes of the plate, and the cerclage cable holes of the plate may be sloped downward from the central longitudinal axis toward the lateral sides of the plate. When the cerclage crimp is positioned within the crimp slot, a crimp spacing may exist within the crimp slot between the flared ends of the crimp and the plate. The bone plate may have a top surface and an opposite, bottom surface contoured to contact a greater trochanter of a proximal femur.

According to one embodiment, a stabilization system for stabilizing a proximal femur having an existing prosthesis may include a plate, a plurality of fasteners, a cerclage crimp, a cerclage cable, and an inserter. The plate has a top surface and an opposite, bottom surface configured to contact a greater trochanter of the proximal femur. The plate includes hooks, fastener openings defined through the plate, a crimp slot having crimp-holding tabs defined along a bottom of the slot, and cerclage cable holes extending through the plate and aligned with the crimp slot. The plurality of fasteners are receivable through the fastener openings and configured to secure the plate to the proximal femur. The cerclage crimp is positionable in the crimp slot such that the cerclage crimp is recessed into the crimp slot, the crimp-holding tabs retain the cerclage crimp therein, and cable holes in the cerclage crimp align with the cerclage cable holes in the plate. The cerclage cable is positionable through the plate and the cerclage crimp to provide circumferential compression to the proximal femur. The inserter is configured to install the plate along the proximal femur.

The stabilization system may include one or more of the following features. The plate may include at least two posterior fastener openings and at least two anterior fastener openings. The posterior fastener openings may be configured to target a lesser trochanter of the proximal femur. The fastener openings may include polyaxial openings having a cone of angulation up to 40 degrees. The inserter may include a shaft and a handle. The shaft may have a distal tip configured to temporarily connect the inserter to the plate. The plate may define a connector hole having threads at its proximal end, and the distal tip of the inserter may include corresponding threads that match with the threads in the connector hole. The plate may define a chamfered edge surrounding a top of the connector hole, and the distal tip of the inserter may include a chamfered edge that bottoms out on the chamfered edge of the plate when the inserter is fully inserted. The plate may define a flat surrounding a top of the connector hole, and the distal tip of the inserter may include a corresponding flat that bottoms out on the flat of the plate when the inserter is fully inserted.

According to one embodiment, a method of installing a periprosthetic plate for stabilizing a proximal femur having an existing prosthesis may include one or more of the following in any suitable order: (a) providing a periprosthetic plate having hooks, a plurality of fastener openings defined through the plate, at least one crimp slot having crimp-holding tabs defined along a bottom of the slot, cerclage cable holes extending through the plate and aligned with the crimp slot, and a threaded connector hole at a proximal end of the plate; (b) temporarily securing an inserter to the plate by threading a tip of the inserter into the threaded connector hole; (c) placing the plate against a proximal femur such that the hooks go around a tip of a greater trochanter of the proximal femur; (d) inserting fasteners through the fastener openings such that at least one of the fasteners targets a lesser trochanter of the proximal femur; (e) positioning a cerclage crimp having cable holes in the crimp slot of the plate; (f) looping a cerclage cable around the proximal femur, through the cerclage cable holes of the plate, and through the cable holes of the cerclage crimp; (g) tensioning the cerclage cable around the proximal femur; and (h) deforming the cerclage crimp to secure the cerclage cable. The plate may be positioned lateral or posterolateral, with the hooks passing through abductor muscles and resting medially to the tip of the greater trochanter. The method may also include: (i) striking a proximal end of the inserter with a mallet to drive the hooks of the plate into soft tissue and/or the proximal femur. The tip of the inserter may be threaded into the threaded connector hole until a shaft of the inserter bottoms out on a beveled of flattened surface around the connector hole.

Also provided are kits for the stabilization systems including periprosthetic bone plates of varying types and sizes, fasteners of varying types and sizes, cerclage cables, K-wires, sutures, instruments, and other components for installing the same.

### BRIEF DESCRIPTION OF THE DRAWINGS

A more complete understanding of the present invention, and the attendant advantages and features thereof, will be more readily understood by reference to the following detailed description when considered in conjunction with the accompanying drawings, wherein:
FIG. 1 shows a periprosthetic plate on the proximal femur with an existing prosthesis according to one embodiment;
FIGS. 2A-2C shows top, bottom, and left views, respectively, of a greater trochanter hook plate according to one embodiment;
FIG. 3 shows a cerclage crimp according to one embodiment;
FIGS. 4A-4B shows the greater trochanter hook plate and cerclage crimp with alternative connector hole options;
FIG. 5 shows a cross-section of the cerclage crimp and greater trochanter hook plate with alignment of the cerclage cable holes according to one embodiment;
FIG. 6 shows application of a cerclage cable through the hook plate and around the proximal femur according to one embodiment;
FIG. 7 shows an inserter instrument including a close-up of attachment to the hook plate at a threaded connector hole according to one embodiment;
FIG. 8 shows a hook plate inserter with a shaft and a handle according to one embodiment;
FIG. 9 shows the shaft of the hook plate inserter according to one embodiment;
FIGS. 10A-10C show isometric, right, and front views, respectively, of the handle of the hook plate inserter according to one embodiment;
FIG. 11 shows the distal tip of the hook plate inserter attachable to the plate including a close-up view of the hook plate inserter distal tip according to one embodiment; and
FIGS. 12A-12B show cross-sectional views of the inserter connected to alternative types of threaded connector holes in the plate.

### DETAILED DESCRIPTION

Embodiments of the disclosure are generally directed to devices, systems, instruments, and methods for promoting healing and stability for bone fractures, particularly periprosthetic fractures. The periprosthetic plates may include hook plates configured to heal fractures around prosthetic joints, such as those near the greater trochanter of the proximal femur. The hook plates may be especially suited for fractures that occur in the context of a total hip replacement where traditional fixation techniques might be compromised due to the presence of the existing prosthesis.

Although the plates are generally described with reference to stabilizing the greater trochanter of the proximal femur, it will be appreciated that the stabilization systems described herein may be used or adapted to be used for the fixation of other areas or other bones as well including the tibia, humerus, clavicle, fibula, radius, ulna, bones of the hand, bones of the feet, or other suitable bone(s) or joint(s). The bone plates may be available in a variety of lengths, widths, and styles based on the anatomy of the patient and types of fractures. The systems may be adapted to secure small or large bone fragments, single or multiple bone fragments, or otherwise secure one or more periprosthetic fractures.

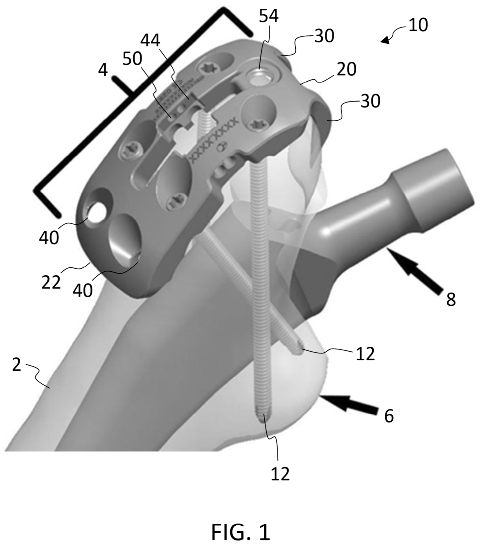

Turning now to the drawing, where like reference numerals refer to like elements, FIG. 1 shows a close-up view of the upper part of the thigh bone or proximal femur 2, which articulates with the pelvis at the hip joint. The proximal femur 2 includes the region of the greater trochanter 4, which is a prominent, palpable bony projection located at the lateral aspect of the proximal femur 2. The proximal femur 2 also includes the region of the lesser trochanter 6, which is a smaller bony projection found on the medial side of the proximal femur 2, just below the neck of the femur 2.

As shown in FIG. 1, the proximal femur 2 may have an existing orthopedic implant, such as an intramedullary prosthesis 8, which may have been previously installed in the context of joint replacement or complex fractures. For example, during a hip replacement, the femoral head and neck of the femur 2 may be replaced with the prosthesis 8 to restore joint function and relieve pain. Similarly, for complex fractures of the femur 2, intramedullary prostheses 8 may be used for fractures involving the femoral neck or intertrochanteric region where traditional fixation devices might not provide sufficient stability. The intramedullary prosthesis 8 may include a stem, inserted into the marrow cavity (intramedullary canal) of the femur 2, and a proximal body configured to replace the head of the femur 2 or to bridge and support the area around the proximal femur 2.

At a later time, a periprosthetic fracture may occur adjacent or around the existing prosthesis 8 necessitating the installation of periprosthetic plate 10 to stabilize the fractured bone around the existing prosthesis 8. For example, the periprosthetic plate 10 may be configured to be placed in the region of the greater trochanter 4 to provide mechanical support and aid in the healing process. The periprosthetic plate 10 may include one or more hooks 30 intended to go around the tip of the greater trochanter 4. The greater trochanter hook plate 10 may be positioned lateral or posterolateral, with the hook(s) 30 passing through the abductor muscles and resting medially to the tip of the greater trochanter 4. The plate 10 may be especially suitable for simple Ag fractures (e.g., fractures located proximal to the prosthetic stem and involving the greater trochanter). If required, the plate 10 may be tensioned to reduce the fracture.

The periprosthetic plates 10 may be available in multiple sizes to match various greater trochanter patient anatomies. These plates 10 may be provided in a number of variations in a surgical tray, which include for example various types, sizes, and configurations. The tray selection may allow for the surgeon to select a desired plate during surgery after opening the wound area and considering the plating needs for the specific patient.

To secure the periprosthetic plate 10, one or more bone fasteners 12 (e.g., screws) may be placed through the plate 10 and into the bone. The fasteners 12 may have nominal screw hole trajectories targeting certain aspects of the bone, for example, better quality or denser bone. In the case of a greater trochanter hook plate, one or more of the fasteners 12 may be targeted into the greater trochanter 4 and toward the region of the lesser trochanter 6. The screws 12 in the greater trochanter hook plate 10 may be positioned to achieve two results. Firstly, the screws 12 must not interfere with the existing prosthesis or intramedullary nail 8. It is important that the additional hardware does not destabilize, dislodge, or damage the existing prosthesis 8. Secondly, the nominal (co-axial with the hole) screw hole trajectories of the two most proximal posterior screw holes preferably target the areas of the proximal femur 2 with the best (densest) bone. This bone is typically around the lesser trochanter 6 and therefore at least two posterior screws 12 may be targeted to the lesser trochanter area 6. The quality of the anterior bone of the greater trochanter 4 can vary according to the fracture and patient, but still can include the placement of anterior screws 12 for additional fixation. The density and bone quality where the screws 12 are anchored significantly affect the strength and longevity of the of the repair.

The bone fasteners 12 may include locking fasteners, non-locking fasteners, or any other suitable fasteners. The fasteners 12 may be cannulated such that they may be guided into place over guide wires or K-wires, for example. The fasteners 12 may comprise bone screws or the like. The fasteners 12 may also include other fasteners or anchors configured to be secured or engaged with bone, such as nails, spikes, staples, pegs, barbs, hooks, or the like. In some embodiments, the fasteners 12 may include fixed and/or variable angle bone screws. The screws 12 may include a head portion and a threaded shaft portion configured to engage bone. In the case of a locking fastener 12, the head portion may include a textured area, such as threads, around its outer surface sized and configured to engage with an opening 40, for example, with corresponding threads or textured area in the opening 40 in order to lock the fastener 12 to the plate 10. In the alternative, for a non-locking fastener 12, the head portion may be substantially smooth and rounded to allow for plate positioning and/or dynamic compression of the bone 2. The fasteners 12 may have a threaded shaft portion configured to secure the plate 10 to the bone.

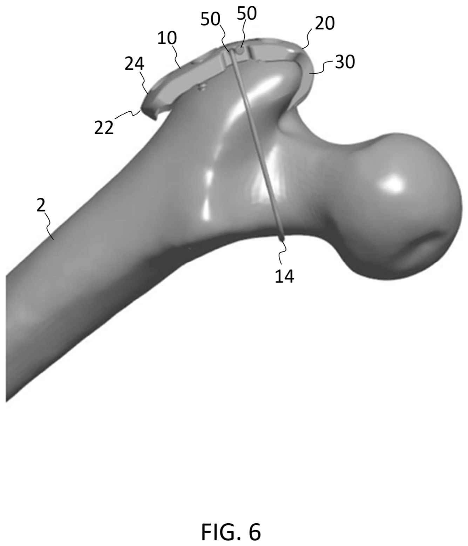

In addition to the bone fasteners 12, a cerclage cable 14 may be used to secure the plate 10 to the femur 2, secure bone fragments, and/or to provide additional stabilization around the femur 2. As best seen in FIG. 6, the cerclage wire or cable 14 may be wrapped or looped around the shaft or neck of the femur 2. The cable 14 may be tensioned to ensure the bone fragments are held firmly without compromising the blood supply to the bone or causing additional trauma. The cerclage cable 14 may be tightened to provide circumferential compression around the bone supplementing the longitudinal stability provided by the plate itself. The cerclage cable 14 may be especially beneficial in cases where the bone integrity is compromised, for example, in osteoporotic patients or cases of complex fractures.

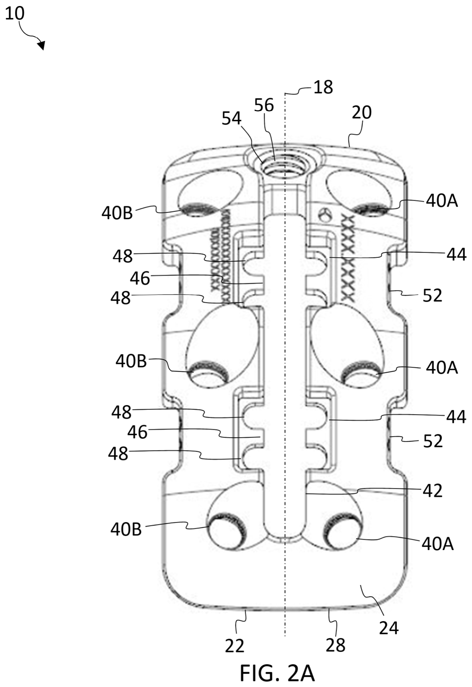

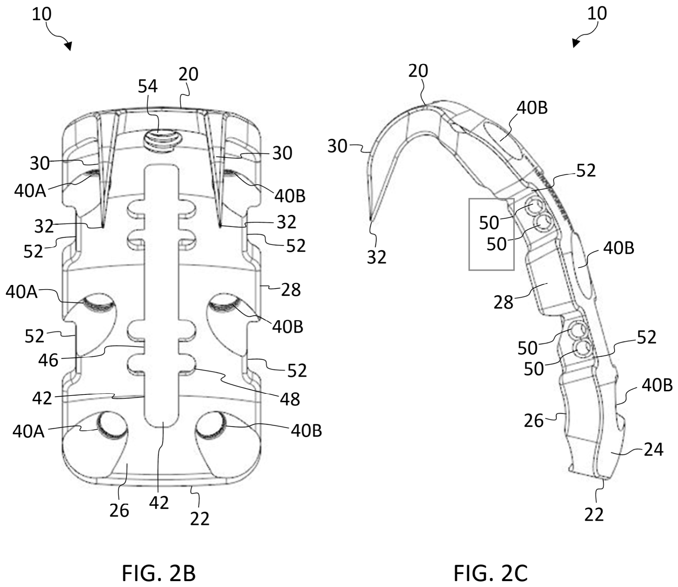

Turning now to FIGS. 2A-2C, periprosthetic plate 10 is shown according to one embodiment. The periprosthetic plate 10 has a body that extends along a central longitudinal axis 18 from a first end or proximal end 20 to a second end or distal end 22. With reference to the human body and components of the system described herein which are intended to be implanted in the human body, the anatomical terms proximal and distal are used to describe relative positions along a limb. Specifically, proximal refers to the end of a limb, bone, or plate that is nearer to the torso where the limb joins the body, while distal denotes the end of a limb, bone, or plate that is farther away from the torso. In this case, the proximal end 20 of the plate 10 may be located near the proximal end of the femur 2, and the distal end 22 of the plate 10 may be located along the shaft of the femur 2, for example, such that the plate 10 covers the greater trochanter region 4 of the femur 2. It will be appreciated that the plate 10 may be located in any suitable location or position as selected by the surgeon.

The periprosthetic plate 10 may have a generally rectangular body including a top surface 24, an opposite bottom surface 26 configured to contact adjacent bone, with sides 28 defined therebetween. The top and bottom surfaces 24, 26 of the plate 10 may be contoured to follow the surface of the bone it is placed against. For example, the plate 10 may have a curvature along axis 18, and the bottom surface 26 may include a concave curvature or pre-contoured geometry having an anatomic contour. In the case of the proximal femur, the bottom surface may be configured to follow the best approximation of the greater trochanter region 4 of the proximal femur 2. The sides 28 of the plate 10 may define a plate thickness, which is minimized. The plate thickness may be reduced across the lateral greater trochanter to reduce prominence and minimize soft tissue irritation. By maintaining a lower profile, the plate 10 also minimizes patient discomfort and can be less visible or palpable through the skin, contributing to better overall patient outcomes in terms of mobility, pain, and satisfaction post-surgery.

The periprosthetic plate 10 may include one or more hooks 30 configured to go around the tip of the greater trochanter region 4. For example, a pair of hooks 30 may be located on the proximal end 20 of the plate 10. The hooks 30 may be symmetrically placed about the centerline 18 of the plate 10 to balance the forces applied to the bone during movement and weight bearing. Each hook 30 may have an attaching portion attached to the proximal end 20 and may terminate as a pointed tip 32 to facilitate penetration into the bone or to securely grip the outer surface of the bone. Each hook tip 32 may narrow in width and/or thickness reducing the overall profile of the hook 30 to form a sharpened point. The tip 32 may create a piercing end that can easily initiate entry into tissue or bone and provide for precise placement of the plate 10. The hooks 30 may be curved such that the tips 32 point generally distally and the curvature matches the anatomical contour of the tip of the greater trochanter 6. The hooks 30 may be configured to pass through the abductor muscles and rest medially to the tip of the greater trochanter 6. The abductor muscles, primarily the gluteus medius and minimus, are key muscles in the lateral hip that function to abduct the thigh (move it away from the body's midline) and stabilize the pelvis during walking. The hooks 30 may be configured to pass through the abductor such that the pathway of hook insertion minimally disrupts these muscles. The hooks 30 may be placed on the inner side of the femur 2 toward the pelvic bone to leverage the dense bone medial to the trochanteric region 6 for better grip and stability. For example, the hooks of the pair of hooks 30 are curved and extend from the proximal end 20 along a curved trajectory having a trajectory component leading towards the distal end 24 of the plate 10 and a trajectory component leading away from the bottom surface 26 of the plate10. In this way, the pointed tip 32 of the hooks 30 is distanced from the bottom surface 26 of the plate 10 and it is closer than to the attaching portion to the distal end 24.

In one embodiment, the plate 10 is contoured to align with the natural shape of the proximal femur 2. The plate 10 includes multiple fastener or screw holes 40 configured to receive the bone fasteners 12. The screw holes 40 may target the fasteners 12 into specific areas of the femur 12, such as the anterior and posterior anatomy of the femur 2 including the lesser trochanter 6. In one embodiment, the plate 10 includes a minimum of four screw holes 40: two posterior holes 40A and two anterior holes 40B. In one embodiment shown in FIGS 2A-2C, three posterior holes 40A may be provided along the posterior side of the plate 10 and three anterior holes 40B may be provided along the anterior side of the plate 10. The two most proximal posterior screw holes 40A may target the area of the lesser trochanter 6 (as best seen in FIG. 1), which is typically denser bone providing a strong anchor for the posterior part of the plate 10. The screws 12 in the two most proximal posterior screw holes 40A may be aimed to converge or point distally and/or medially into the lesser trochanter 6 (e.g., the proximal-most screw 12 aims more distally and the second-most proximal screw 12 aims more medially into the region of the lesser trochanter 6). The anterior holes 40B may target the anterior side of the femur 2 to help stabilize the anterior side of the plate 10, thereby complementing the posterior fixation to distribute loads more evenly across the fracture site. The dual targeting approach uses the anatomical strengths of the lesser and greater trochanters 4, 6 to provide balanced stability, distributing mechanical stress and reducing the risk of fixation failure. The remaining screw holes 40A, 40B may target any other suitable areas of bone or may allow the surgeon to select the ideal trajectory. The plates 10 may comprise any suitable number of screw holes in any suitable configuration. These screw holes allow surgeons flexibility for fastener placement based on preference, anatomy, and fracture location.

In one embodiment, the screw holes 40 may include polyaxial screw holes, which allow for angulation of the screws 12 to accommodate a wide variety of anatomy and fracture patterns. An upper portion of the hole 40 may be tapered, beveled, or angled to accommodate insertion of the screw 12 at the desired degree of angulation. In one embodiment, all screw holes 40 in the plate are polyaxial screw holes (e.g., 3.5mm), which allow for up to a 40-degree cone of angulation. The polyaxial holes 40 may be locking holes with threads or a textured area configured to deform and/or engage with the screw head. Additional details on these and other types of openings are provided in further detail in U.S Patent No. 11,432,857, which is incorporated by reference herein in its entirety for all purposes.

The plate 10 defines a central slot 42, which may be aligned with the middle or central axis 18 of the plate 10. The central slot 42 may include a narrow linear slot that runs longitudinally along the centerline of the plate 10 between the screw holes 40. The central slot 42 defines one or more crimp slots 44 sized and dimensioned to receive a cerclage crimp 60 therein. The crimp slots 44 may define a generally quadrilateral recess, such as a square or rectangular shaped recess. The bottom surface 26 of the plate 10 may extend along the bottom of the crimp slot 44 to form one or more crimp-holding tabs 46. Alternatively, the crimp-holding tabs 46 may project into the crimp slot 44 to support the cerclage crimp 60 such that the cerclage crimp 60 is recessed in the crimp slot 44 while the top of the cerclage crimp 60 remains generally flush with the top surface 24 of the plate 10. Each crimp-holding tab 46 may include a projection defined between two semi-circular, obround, or elliptical cuts 48. In one embodiment, each crimp slot 44 includes a pair of crimp-holding tabs 46 centrally located in the crimp slot 44 and pointing inward toward the centerline 18 and toward one another. As best seen in FIG. 1, the hook plate 10 may include a single crimp slot 44 located in the area between the proximal-most and central screw holes 40. Alternatively, as shown in FIGS. 2A-2C, the plate 10 may include two crimp slots 44, one located in the area between the proximal-most and central screw holes 40 and a second located in an area between the central and distal-most screw holes 40. It will be appreciated that the crimp slot(s) 44 may be located at strategic points along the plate 10 to securely hold the cerclage crimps 60, which secures the cerclage cable 14 therein.

As best seen in FIG. 2C, the plate 10 defines cerclage cable holes 50 between the crimp slot 44 and the lateral sides 28 of the plate 10. Each cerclage cable hole 50 may include a cylindrical hole defined through the body of the plate 10. The cerclage cable hole 50 is a throughout hole opened at opposite sides thereof respectively at the crimp slot 44 and lateral sides 28 of the plate 10. There may be a minimum of one cerclage cable hole set (e.g., four holes each) per plate size. Each cable hole set can accommodate a single cerclage cable 14. On either side of each crimp slot 44, two parallel cable holes 50 may be aligned on one side and two parallel cable holes 50 may be aligned on the opposite side of the slot 44. The cable holes 50 may terminate into a recessed cut 52 along the lateral sides 28 of the plate 10. The recessed cuts 52 may define a flat bottom in the region of the cable holes 50 with sloped or angled ends that connect to the lateral sides 28. In other words, the lateral sides 28 of the plate 10 may be indented along the recessed cuts 52, thereby shortening the span of the cable holes 50 through the plate 10. The recessed cuts 52 on the sides 28 of the plate 10 help to decrease the channel length that the cables 14 need to pass through and therefore, may help to guide and facilitate cable passage through the plate 10.

The hook plate 10 may define at least one connector or attachment interface 54 for attaching an instrument, such as inserter 100, which helps to install the plate 10 and drive the hooks 30 of the plate 10 into the soft tissue and/or bone during installation. The attachment interface 54 may include a cylindrical hole with a threaded portion 56 that engages with the inserter 100. The connector hole 54 may be located at the most proximal portion of the plate 10, and may be centrally positioned along the centerline 18. It will be appreciated that the connector hole 54 may be located at any suitable location, which does not interfere with the screw trajectories. It will also be appreciated that another attachment interface may be used to attach a suitable instrument to the plate 100 for installation.

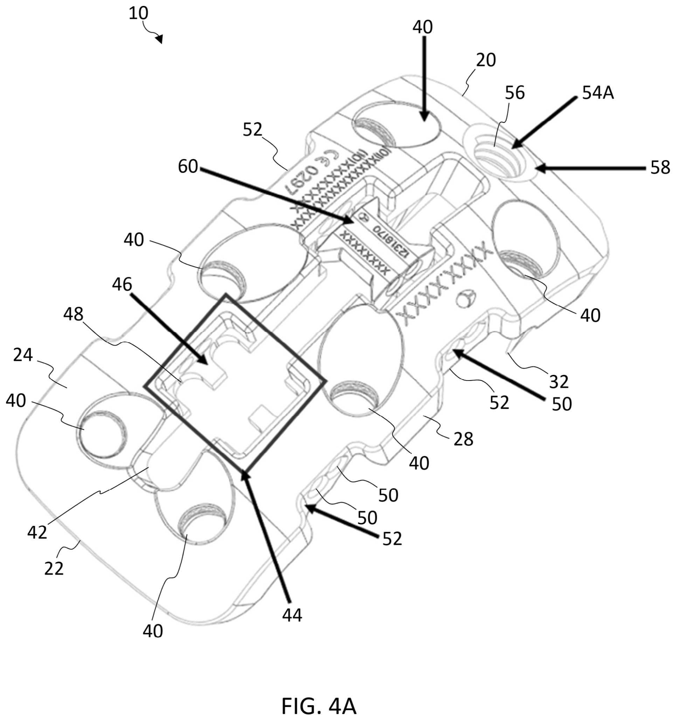

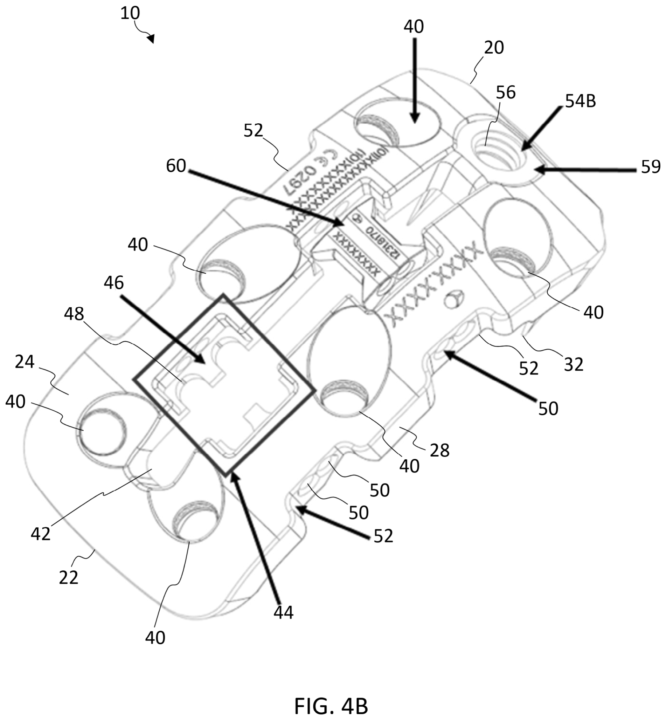

In one embodiment, the connector hole 54 may feature a hole with an M6x1.0 thread (e.g., nominal diameter of the thread is 6 millimeters and the pitch of the thread is 1.0 millimeter apart from the next). The threaded hole 54 may include any suitable nominal diameter and pitch for suitable attachment to the hook plate inserter 100. With further emphasis on FIGS. 4A-4B, the connector hole 54 may include different attachment options. In FIG. 4A, a first connector hole type 54A is shown with a chamfered edge 58 surrounding the top of the threaded hole. When the inserter 100 is attached to the hook plate 10, the hook plate inserter 100 bottoms out, after a minimum of two full threads of engagement, on the chamfered edge 58 surrounding the top of the threaded hole. In FIG. 4B, a second connector hole type 54B is shown with a flat 59 surround the top of the threaded hole. In this case, the hook plate inserter 100 bottoms out on the flat 59 around the threaded hole. Both options create a robust and rigid connection between the inserter 100 and the hook plate 10.

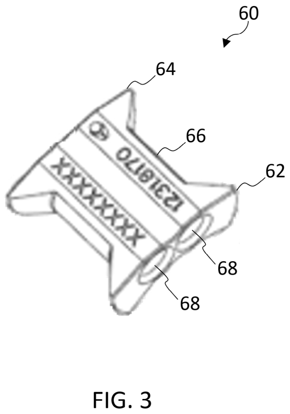

As best seen in FIG. 3, a cerclage crimp 60 is shown according to one embodiment. The cerclage crimp 60 is configured to fit into the crimp slot 44 and secure the cerclage cable 14 to the construct. The cerclage crimp 60 may extend from a first end 62 to a second end 64 with a central body 66 that narrows toward the middle resembling a bowtie or hourglass form. The ends 62, 64 of the crimp 60 may be flared or angled outward from the narrowed central body 66. The crimp ends 62, 64 may taper sharply to pointy ends, which help to align and position the crimp 60 into the designated crimp slot 44. The cerclage crimp 60 defines one or more through holes or cable holes 68 that extend between the ends 62, 64 of the crimp 60. The cable holes 68 may include cylindrical openings sized and dimensioned to receive the cerclage cable 14 therethrough. The crimp holes 68 may include a pair of holes aligned in parallel to one another. The pair of crimp holes 68 may be configured to receive a single cerclage cable 14.

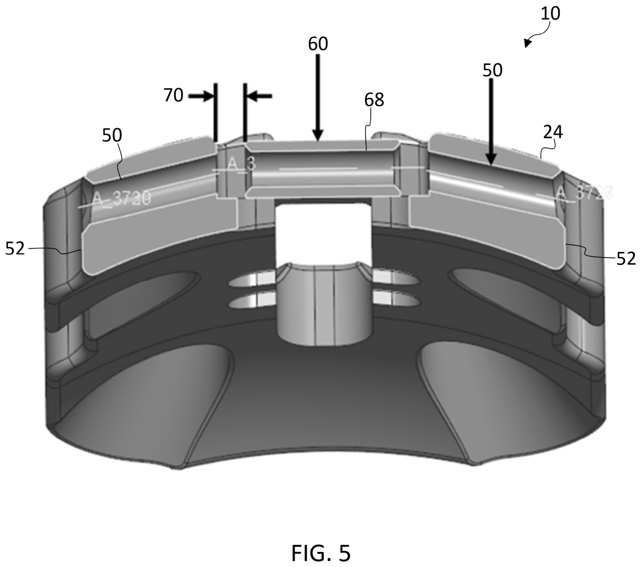

With further emphasis on FIG. 5, when the cerclage crimp 60 is positioned in the crimp slot 44, the cable holes 68 of the cerclage crimp 60 align with the cable holes 50 in the plate 10. The crimp slots 44 include crimp-holding tabs 46 at the bottom or within the slots 44, which allow the cerclage crimp 60 to sit inside of the plate 10 and align the cable holes 50 of the plate 10 with the crimp holes 68 of the cerclage crimp 60. The cable holes 50 in the plate 10 may be offset toward the top surface 24 of the plate 10 such that more plate material exists on the bottom portion of the plate 10, which may provide increased strength and stability to the plate 10. The diameter of the crimp holes 68 may be the same or similar to the diameter of the cable holes 50. The cable holes 50 in the greater trochanter hook plate 10 may be angled or sloped slightly downwards from the middle of the plate 10. In other words, the cable holes 50 of the plate 10 may be sloped from the central longitudinal axis downward toward the lateral sides of the plate 10. When the cerclage cable 14 is inserted, it can travel up through a first cable hole 50 on one side of the plate 10, horizontally through the cable hole 68 in the cerclage crimp 60, and then travel downward through the second cable hole 50 on the opposite side of the plate 10 (e.g., before or after encircling the bone). The angled holes 50 in the plate 10 may help to minimize overall plate thickness as much as possible in order to minimize potential soft tissue irritation.

The cerclage crimp 60 may fit within the crimp slot 44 such that a crimp spacing 70 exists between the ends 62, 64 of the crimp 60 and the walls of the plate 10 defining the cerclage holes 50. The spacing 70 may help to evenly distribute stress induced by tensioning of the cerclage cable 14, preventing localized stress points. In addition, the crimp spacing 70 may facilitate slight adjustments in positioning of the crimp 60 during tensioning of the cerclage cable 14.

The cable(s) 14 may be passed through the cerclage crimp(s) 60 which lay recessed along the centerline of the plate 10, through the cerclage holes 50 in the plate 10, and looped around the femur 2. Once tensioned, the crimp(s) 60 can be deformed to secure the cable(s) 14 and maintain the tensioning. The crimp(s) 60 can be mechanically compressed, for example, with a crimping tool that applies pressure to the crimp and deforms the crimp 60 by squeezing it tightly against the cable 14. The crimp 60 may be squeezed in the area of the narrowed central body 66 to secure the cable 12, preventing it from slipping or moving and providing adequate stabilization to the bone.

The bone plates 10 may be comprised of titanium, stainless steel, cobalt chrome, carbon composite, plastic or polymer-such as polyetheretherketone (PEEK), polyethylene, ultra high molecular weight polyethylene (UHMWPE), resorbable polylactic acid (PLA), polyglycolic acid (PGA), combinations or alloys of such materials or any other appropriate material that has sufficient strength to be secured to and hold bone, while also having sufficient biocompatibility to be implanted into a body. Similarly, the fasteners 12 and cables 14 may be comprised of titanium, cobalt chrome, cobalt-chrome-molybdenum, stainless steel, tungsten carbide, combinations or alloys of such materials or other appropriate biocompatible materials. Although the above list of materials includes many typical materials out of which components are made, it should be understood that parts comprised of any appropriate material are contemplated.

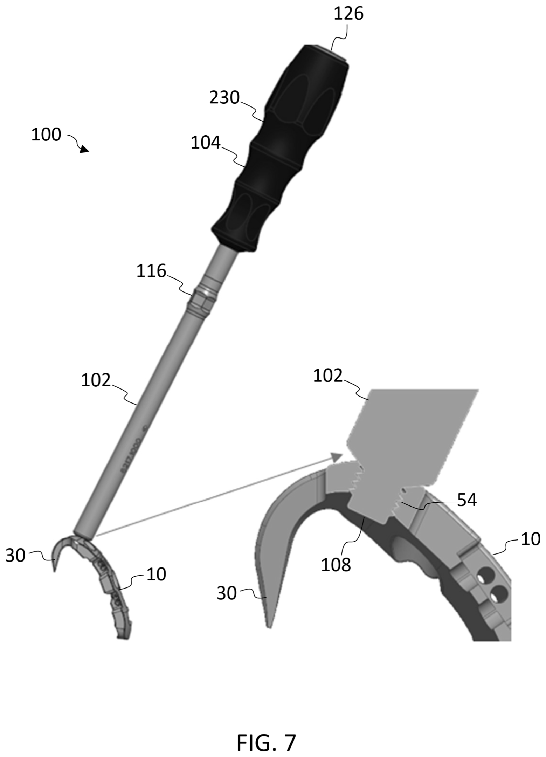

Turning now to FIG. 7, a hook plate inserter 100 is shown according to one embodiment. The hook plate inserter 100 may be attached to the plate 100 for installation. The hook plate inserter 100 may be temporarily coupled to the plate 10 at the attachment interface or connector hole 54. Hook plate 10 may be positioned in the patient with the hook plate inserter 100. The inserter 100 may be used with the hook plate 10 to help drive the hooks 30 of the plate 10 into soft tissue and/or bone. Most other trauma plates sit on little to no soft tissue and do not penetrate bone and therefore, they do not require an inserter for plate positioning. The hook plate inserter 100 may be used as a joystick and/or may be malleted on to seat the hook plate 10 in the desired location. The hook plate inserter 100 may be used to insert the hook plate 10 properly onto the greater trochanter and drive the hooks 30 into the proper location.

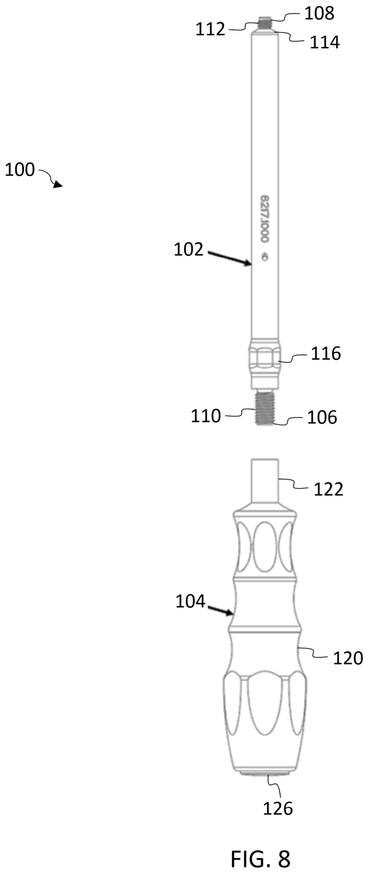

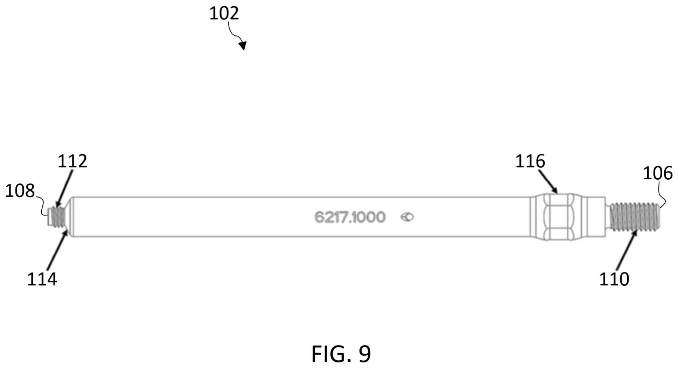

Several current hook plate inserters are bulky in size and/or complex in design. As best seen in FIG. 8, the hook plate inserter 100 is streamlined to include two components: a shaft 102 and a handle 104 aligned along a central tool axis. With further emphasis on FIG. 9, the shaft 102 may have a cylindrical body that extends from a proximal end 106 to a distal tip 108. The proximal end 106 of the shaft 102 may include a male thread 110, which threads into the handle 104 during manufacturing assembly. The distal tip 108 includes thread(s) 112 that thread into the connector hole 54 in the plate 10. The shaft 102 may include an angled or chamfered edge 114 that transitions to the distal tip 108 and reduces the diameter from the body of the shaft 102 to the threaded portion 112 of the distal tip 108. The shaft 102 may also include flats 116 designed to maximize leverage and provide a secure grip for a wrench, for example. The flats 116 may be positioned radially about the shaft 102, for example, toward the proximal end 106, to allow the inserter 100 to be easily un-torqued from the plate 10 after use if needed.

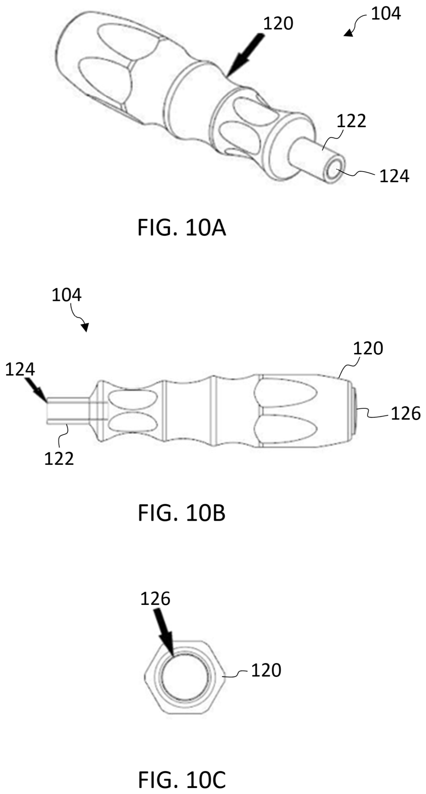

With further emphasis on FIGS. 10A-10C, the handle 104 includes a handle grip 120 and a sleeve 122 defining a threaded opening 124 for receiving the threaded end 110 of the shaft 102. The threaded opening 124 may include a female thread to mate with the shaft 102 of the hook plate inserter 100 in manufacturing assembly. The handle grip 120 may be ergonomic, for example, with a scalloped and contoured handle, which allows for comfortable grip of the instrument 100 and prevents slipping when in a surgical environment. The handle grip 120 gives the user a convenient and comfortable handling location, for example, made of a soft silicone material. The proximal end of the handle 104 may include an impaction location 126, which may be impacted by another instrument. The impaction location 126 may include a durable and large steel impaction area, for example, which may be struck by a mallet or other instrument to seat the hook plate 10 into the desired location.

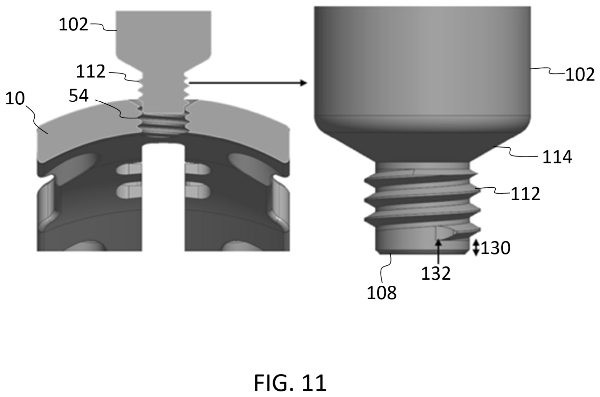

With further emphasis on FIG. 11, the hook plate inserter 100 may be secured to any hook plate 100 by aligning the threaded tip 112 to the threaded hole 54 in the hook plate 10 and then threading the tip 112 into the plate 10 until the inserter shaft 102 bottoms out on the plate 10. In one embodiment, the tip 108 of the hook plate inserter 100 has a small portion at the distal-most end that is unthreaded 130. The unthreaded section 130 functions as a pilot diameter to facilitate properly orienting the hook plate inserter 100 within the hook plate connection hole 54 and helps to prevent cross-threading. Additionally, the tip 108 may have a blunt start thread 132 (e.g., a Higbee cut) initiating the beginning of thread 112. The blunt start thread 132 may also help to prevent cross-threading between the plate 10 and the inserter 100.

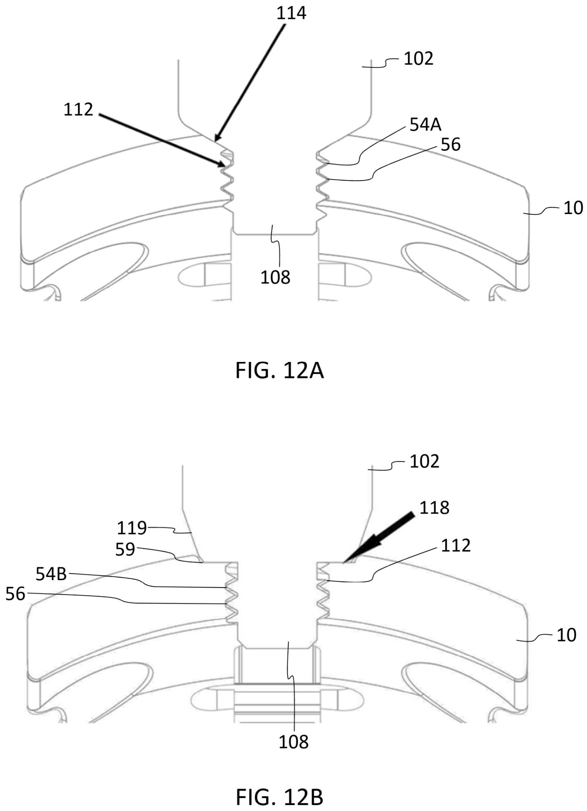

Depending on the type of connector hole type in the plate 10, the distal tip 108 of the inserter 100 may be modified to account for the inserter connection interface with the plate 10. As best seen in FIG. 12A, the shaft 102 of inserter 100 may include chamfered edge 114 which bottoms out on the mating chamfered edge 58 of connector hole 54A on the proximal end of the hook plate 10. In the alternative shown in FIG. 12B, the shaft 102 of inserter 100 may include a flat surface 118 that transitions into a chamfered portion 119 between the distal tip 108 and the main body of the shaft 102. In this case, the male threads 112 of the inserter 100 thread into the female threads 56 of the connector hole 54B until the flat surface 118 of the shaft 102 bottoms out on the flat surface 59 of the connector hole 54B of the hook plate 10. In this manner, the inserter 100 is securely but temporarily attached to the hook plate 10 for installation.

The periprosthetic hook plate portfolio may include three types of hook plates: the greater trochanter hook plate, a hook attachment plate, and a proximal femur hook plate. All three styles of plates may have the same proximal attachment interface, and therefore may all be used with the same hook plate inserter.

The greater trochanter hook plates may include several enhancements to improve its usability and effectiveness. Firstly, the thickness of the plate across the lateral greater trochanter may be minimized, reducing its prominence and potential for soft tissue irritation. Secondly, the screw trajectories may be optimized to target the areas of densest bone, ensuring stronger fixation. Additionally, the placement of screws may be carefully planned to avoid any interference with existing prosthetic components within the body. Moreover, the hook plate may optionally include a portion of the plate distal to the vastus ridge is not connected. This separation enables the anterior and posterior segments of the plate to be individually contoured to match their respective anatomies. This allows for a more precise adaptation to the respective anatomical features of the greater trochanter area.

The hook plate inserter is configured to enhance its functionality and reliability when installing the plate in the patient. The inserter may include the threaded tip designed for direct insertion into the hook plate, thereby ensuring a secure and robust connection with all hook plates. For example, the inserter may feature the chamfered or flattened surface that aligns and matches with the corresponding surface of the threaded hole on the hook plate, creating a secure, robust-feeling connection. Additionally, the inserter can minimize the risk of cross-threading with the threaded hole in the plate by incorporating a pilot diameter before the start of the threads and a Higbee cut at the thread start, which together facilitate smoother and more accurate threading.

It will be further understood that various changes in the details, materials, and arrangements of the parts which have been described and illustrated in order to explain the nature of this invention may be made by those skilled in the art without departing from the scope of the invention as expressed in the claims. One skilled in the art will appreciate that the embodiments discussed above are non-limiting. It will also be appreciated that one or more features of one embodiment may be partially or fully incorporated into one or more other embodiments described herein.

The invention could be defined inter alia by the following examples:
1. A periprosthetic bone plate system comprising: a bone plate extending along a central longitudinal axis from a proximal end to a distal end configured to sit on bone, a pair of hooks extending from the proximal end, the plate defines a crimp slot having crimp-holding tabs, and cerclage cable holes extending through the plate from the crimp slot to lateral sides of the plate; a cerclage crimp positionable in the crimp slot such that the crimp-holding tabs retain the cerclage crimp therein, the cerclage crimp having a narrowed body with flared ends, the cerclage crimp defining a pair of cable holes between the flared ends; and a cerclage cable positionable through the plate and the cerclage crimp to secure the plate to the bone.
2. The system of Example 1, wherein the crimp-holding tabs include a pair of crimp-holding tabs on opposite sides of the crimp slot pointing inward toward one another.
3. The system of Example 1, wherein each crimp-holding tab includes a projection defined between two semi-circular cuts.
4. The system of Example 1, wherein the crimp slot defines a quadrilateral recess sized and dimensioned to receive the cerclage crimp.
5. The system of Example 1, wherein the plate defines a central linear slot aligned with the central longitudinal axis of the plate, and the crimp slot is positioned along the central linear slot.
6. The system of Example 1, wherein the cerclage cable holes include a set of cerclage cable holes with two parallel cable holes on one side and two parallel cable holes on an opposite side of the crimp slot.
7. The system of Example 1, wherein when the cerclage crimp is positioned within the crimp slot, the cable holes of the cerclage crimp align with the cerclage cable holes of the plate, and the cerclage cable holes of the plate are sloped downward from the central longitudinal axis toward the lateral sides of the plate.
8. The system of Example 1, wherein when the cerclage crimp is positioned within the crimp slot, a crimp spacing exists within the crimp slot between the flared ends of the crimp and the plate.
9. The system of Example 1, wherein the bone plate has a top surface and an opposite, bottom surface contoured to contact a greater trochanter of a proximal femur.
10. A stabilization system for stabilizing a proximal femur having an existing prosthesis, the system comprising:
   a plate having a top surface and an opposite, bottom surface configured to contact a greater trochanter of the proximal femur, the plate including hooks, fastener openings defined through the plate, a crimp slot having crimp-holding tabs defined along a bottom of the slot, and cerclage cable holes extending through the plate and aligned with the crimp slot;
   a plurality of fasteners receivable through the fastener openings and configured to secure the plate to the proximal femur;
   a cerclage crimp positionable in the crimp slot such that the cerclage crimp is recessed into the crimp slot, the crimp-holding tabs retain the cerclage crimp therein, and cable holes in the cerclage crimp align with the cerclage cable holes in the plate;
   a cerclage cable positionable through the plate and the cerclage crimp to provide circumferential compression to the proximal femur; and
   an inserter configured to install the plate along the proximal femur.
11. The system of Example 10, wherein the plate includes at least two posterior fastener openings and at least two anterior fastener openings, wherein the posterior fastener openings are configured to target a lesser trochanter of the proximal femur.
12. The system of Example 10, wherein the fastener openings include polyaxial openings having a cone of angulation up to 40 degrees.
13. The system of Example 10, wherein the inserter includes a shaft and a handle, the shaft having a distal tip configured to temporarily connect the inserter to the plate.
14. The system of Example 13, wherein the plate defines a connector hole having threads at its proximal end, and the distal tip of the inserter includes corresponding threads that match with the threads in the connector hole.
15. The system of Example 14, wherein the plate defines a chamfered edge surrounding a top of the connector hole, and the distal tip of the inserter includes a chamfered edge that bottoms out on the chamfered edge of the plate when the inserter is fully inserted.
16. The system of Example 14, wherein the plate defines a flat surrounding a top of the connector hole, and the distal tip of the inserter includes a corresponding flat that bottoms out on the flat of the plate when the inserter is fully inserted.
17. A method of installing a periprosthetic plate for stabilizing a proximal femur having an existing prosthesis, the method comprising:
   providing a periprosthetic plate having hooks, a plurality of fastener openings defined through the plate, at least one crimp slot having crimp-holding tabs defined along a bottom of the slot, cerclage cable holes extending through the plate and aligned with the crimp slot, and a threaded connector hole at a proximal end of the plate;
   temporarily securing an inserter to the plate by threading a tip of the inserter into the threaded connector hole;
   placing the plate against a proximal femur such that the hooks go around a tip of a greater trochanter of the proximal femur;
   inserting fasteners through the fastener openings such that at least one of the fasteners targets a lesser trochanter of the proximal femur;
   positioning a cerclage crimp having cable holes in the crimp slot of the plate;
   looping a cerclage cable around the proximal femur, through the cerclage cable holes of the plate, and through the cable holes of the cerclage crimp;
   tensioning the cerclage cable around the proximal femur; and
   deforming the cerclage crimp to secure the cerclage cable.
18. The method of Example 17, wherein the plate is positioned lateral or posterolateral, with the hooks passing through abductor muscles and resting medially to the tip of the greater trochanter.
19. The method of Example 17 further comprising striking a proximal end of the inserter with a mallet to drive the hooks of the plate into soft tissue and/or the proximal femur.
20. The method of Example 17, wherein the tip of the inserter is threaded into the threaded connector hole until a shaft of the inserter bottoms out on a beveled of flattened surface around the connector hole.

## Claims

1. A periprosthetic bone plate system comprising:
a bone plate extending along a central longitudinal axis from a proximal end to a distal end configured to sit on bone, a pair of hooks extending from the proximal end, the plate defines a crimp slot having crimp-holding tabs, and cerclage cable holes extending through the plate from the crimp slot to lateral sides of the plate;
a cerclage crimp positionable in the crimp slot such that the crimp-holding tabs retain the cerclage crimp therein, the cerclage crimp having a narrowed body with flared ends, the cerclage crimp defining a pair of cable holes between the flared ends; and
a cerclage cable positionable through the plate and the cerclage crimp to secure the plate to the bone.

2. The system of claim 1, wherein the crimp-holding tabs include a pair of crimp-holding tabs on opposite sides of the crimp slot pointing inward toward one another.

3. The system of claim 1 or 2, wherein each crimp-holding tab includes a projection defined between two semi-circular cuts.

4. The system of any one of the preceding claims, wherein the crimp slot defines a quadrilateral recess sized and dimensioned to receive the cerclage crimp.

5. The system of any one of the preceding claims, wherein the plate define a central linear slot aligned with the central longitudinal axis of the plate, and the crimp slot is positioned along the central linear slot.

6. The system of any one of the preceding claims, wherein the cerclage cable holes include a set of cerclage cable holes with two parallel cable holes on one side and two parallel cable holes on an opposite side of the crimp slot.

7. The system any one of the preceding claims, wherein when the cerclage crimp is positioned within the crimp slot, the cable holes of the cerclage crimp align with the cerclage cable holes of the plate, and the cerclage cable holes of the plate are sloped downward from the central longitudinal axis toward the lateral sides of the plate.

8. The system of any one of the preceding claims, wherein when the cerclage crimp is positioned within the crimp slot, a crimp spacing exists within the crimp slot between the flared ends of the crimp and the plate.

9. The system of any one of the preceding claims, wherein the bone plate has a top surface and an opposite, bottom surface contoured to contact a greater trochanter of a proximal femur.

10. A stabilization system for stabilizing a proximal femur having an existing prosthesis, the system comprising:
a plate having a top surface and an opposite, bottom surface configured to contact a greater trochanter of the proximal femur, the plate including hooks, fastener openings defined through the plate, a crimp slot having crimp-holding tabs defined along a bottom of the slot, and cerclage cable holes extending through the plate and aligned with the crimp slot;
a plurality of fasteners receivable through the fastener openings and configured to secure the plate to the proximal femur;
a cerclage crimp positionable in the crimp slot such that the cerclage crimp is recessed into the crimp slot, the crimp-holding tabs retain the cerclage crimp therein, and cable holes in the cerclage crimp align with the cerclage cable holes in the plate;
a cerclage cable positionable through the plate and the cerclage crimp to provide circumferential compression to the proximal femur; and
an inserter configured to install the plate along the proximal femur.

11. The system of claim 10, wherein the plate includes at least two posterior fastener openings and at least two anterior fastener openings, wherein the posterior fastener openings are configured to target a lesser trochanter of the proximal femur.

12. The system of claim 10 or 11, wherein the fastener openings include polyaxial openings having a cone of angulation up to 40 degrees.

13. The system of any one of claims 10 to 12, wherein the inserter includes a shaft and a handle, the shaft having a distal tip configured to temporarily connect the inserter to the plate.

14. The system of any one of claims 10 to 13, wherein the plate defines a connector hole having threads at its proximal end, and the distal tip of the inserter includes corresponding threads that match with the threads in the connector hole.

15. The system of claim 14, wherein the plate defines a chamfered edge surrounding a top of the connector hole, and the distal tip of the inserter includes a chamfered edge that bottoms out on the chamfered edge of the plate when the inserter is fully inserted and wherein preferably the plate defines a flat surrounding a top of the connector hole, and the distal tip of the inserter includes a corresponding flat that bottoms out on the flat of the plate when the inserter is fully inserted.
